Europäisches Patentamt

European Patent Office

Office européen des brevets

(11) Publication number: **0 242 069**
**A2**

# EUROPEAN PATENT APPLICATION

(21) Application number: 87302568.8

(22) Date of filing: 25.03.87

(51) Int. Cl.⁴: **B01D 13/04** , B01D 53/22 ,
A61L 15/01 , C08J 7/04

(30) Priority: 14.04.86 US 851990

(43) Date of publication of application:
21.10.87 Bulletin 87/43

(84) Designated Contracting States:
DE FR GB

(71) Applicant: DOW CORNING CORPORATION
3901 S. Saginaw Road
Midland Michigan 48640(US)

(72) Inventor: Cabasso, Israel
131 Buckingham Avenue
Syracuse New York, 13210(US)
Inventor: Fearon, Frederick W.G.
4801 Oak Ridge
Midland Michigan, 48640(US)

(74) Representative: Laredo, Jack Joseph et al
Elkington and Fife High Holborn House 52/54
High Holborn
London, WC1V 6SH(GB)

(54) **Semipermeable composite membranes produced from silicone water based emulsions applied to porous substrates.**

(57) The invention relates to the formation of semipermeable composite membranes produced by applying a curable silicone (polyorganosiloxane) water based micro or macro emulsion to a porous substrate, evaporating the aqueous components of the emulsions, and allowing the emulsified siloxane particles to coalesce and to crosslink to form a cured film on the porous substrate. Because the curable silicone water based emulsion is essentially free of organic solvent it can be applied to solvent sensitive natural and synthetic substrates. The composite membranes produced are useful for the separation of fluids.

Fig. 1

SEPARATION FACTOR $O_2/N_2$ VS. PRESSURE FOR COMPOSITE MEMBRANE

A: ACTUAL SEPARATION VS. PRESSURE
(DOWNSTREAM PRESSURE 1.ATM)
B: MAXIMUM ACTUAL SEPARATION (UPSTREAM
PRESSURE 20PSI AIR, DOWNSTREAM PRESSURE 5-8 mm Hg)
C: AVERAGE RATIO OF OXYGEN TO NITROGEN PERMEABILITY
($P_{O_2}/P_{N_2}$) IN 10-50 PSI PRESSURE RANGE

SEPARATION FACTOR $(O_2/N_2)$

PRESSURE (PSI ABOVE AMBIENT)

# SEMIPERMEABLE COMPOSITE MEMBRANES PRODUCED FROM SILICONE WATER BASED EMULSIONS APPLIED TO POROUS SUBSTRATES

The present invention relates to the discovery that a substantially continuous film of a curable polyorganosiloxane can be formed over the surface of a porous membrane, such as a polysulfone membrane, without destruction or degradation of the porous membrane, if the curable polyorganosiloxane is applied as an aqueous emulsion, the aqueous component of the emulsion removed and the polyorganosiloxane cured.

One of the key difficulties in the application of coatings of films to plastic membranes and substrates is the destruction or degradation of the target membrane or substrate by the solvent medium in which the desired coating or film is borne. For example, asymmetric cellulose acetate, a popular semipermeable membrane material, cannot be coated with a topcoat which is dissolved or carried in hexane because of the destructive effect the hexane will have on the cellulose acetate porous structure. Other solvents which dissolve polymeric materials used in producing permeable and semipermeable membranes are toluene, xylene, isopropyl alcohol, and many ketones. In the current state of the art, one is very limited in solvent usage (both from ecologic and also substrate compatibility viewpoints) for application of coatings.

One area in which the problems of solvent sensitivity of substrates are particularly acute is the preparation of composite membranes or "sandwich" membranes in which two or more dissimilar films are deposited on top of one another for the ultimate purpose of producing a semipermeable membrane with gas or liquid separation capabilities. The difficulty arises when one or more of the polymer films already in the "sandwich" or composing the substrate, exhibit sensitivity to, or cannot be wet out by, the solvent medium needed to dissolve and/or carry the next-to-be-applied film forming polymer. The results of such attempts to coat an incompatible or sensitive substrate include nonhomogeneous coatings and degradation of the substrate, which renders the resultant "sandwich" membrane unsuitable for the separation of gases and/or liquids.

Gas separation has many potential applications in industry and medicine. Currently, devices that utilize combustion energy, such as automobile engines and heating appliances, are designed to operate on the basis that air contains approximately 20% concentration of oxygen. Combustion efficiency is increased, and environmental pollution decreased, if air with an elevated oxygen concentration is supplied. Elevation of the oxygen concentration of air is also useful for respiratory therapy, in treating long abnormalities in premature infants, and in treating skin wounds.

The development of fluid (i.e., gas and liquid) separation membranes has included homopolymerized materials formed into a planar film, laminated films, composite films, and hollow fibers. The fundamental problem in the development of a separation membrane is the production of a polymer film with good gas transport properties, i.e., high permeability coefficient, yet which retains a degree of selectivity.

It is an often cited generalization in selecting a material for membrane separations, that polymers displaying high selectivity for one component of a mixture often are of low permeability while highly permeable polymers are less discriminating between components of a mixture.

Examples of oxygen and nitrogen permeability coefficients and ideal separation factors for a number of polymers are illustrated in Table I.

## TABLE I

### Permeability Coefficients and Separation Factors of Polymers Toward Nitrogen and Oxygen in Air

$$\bar{P} = \frac{cm^3 \ cm}{cm^2 \cdot sec \cdot cm \ Hg} \times 10^{10}$$

| Polymer | $\bar{P}_{N_2}$ | $\bar{P}_{O_2}$ | Ideal SF |
|---|---|---|---|
| Silicone rubber | 281 | 605 | 2.15 |
| Polyphenylene-oxide (PPO) | 3.81 | 15.8 | 4.15 |
| Polystyrene | 0.83 | 2.5 | 3.01 |
| Polycarbonate | 0.3 | 1.4 | 4.67 |
| Polysulfone | 0.24 | 1.3 | 5.41 |

($\bar{P}_{N_2}$ $\bar{P}_{O_2}$ are the permeability coefficients of nitrogen gas and oxygen gas, respectively, and "Ideal SF" is the ideal separation factor.)

Table I shows that $\bar{P}$ varies over four orders of magnitude between polymers, however, all ideal separation factors (SF's) are of the same order. Likewise it is apparent that, although considerable overlap exists, there is a correlation between low permeability and high selectivity and vice versa. One of the reasons for this observation is the influence of the physical state of the polymer on the diffusion process. Table I also shows that while polysiloxanes have a very high relative permeability, they exhibit a separation factor of approximately half that of polycarbonate, polysulfone or poly(phenylene oxide).

Even though a true change of state is not involved, polymers are classified as existing in either a glassy or a rubbery state. Cooling a polymer from a melt results in a material that becomes first rubbery and then, as the temperature is lowered further, a hard and brittle glassy material. The temperature at which a polymer is transformed from a rubbery to a glassy material is the glass transition temperature, Tg. Below Tg, insufficient thermal energy is available for other than short range cooperative movements of polymer chain segments. Above Tg, sufficient energy is available to enable relatively long segments of the polymer chain to move. In order to accommodate the movement of long chain segments, an increase in the free volume of the polymer is necessary. In addition to accommodating chain segments, this free volume may be filled by gaseous penetrants. In rubbery polymers, the free volume should be regarded as a dynamic system in which openings in the polymer matrix are continually being created and filled by chain movements. Glassy polymers, on the other hand, contain much less free volume than do rubbery polymers, and openings in the matrix are relatively fixed in space. Because the total free volume is much less for glassy polymers, permeability tends to be lower, while a narrower distribution of openings in the polymer matrix enables these polymers to more readily discriminate between molecules of different sizes.

In designing a permeation process for both the industrial separation of gases or liquids and medical applications, the major concerns are product purity and rate of production. In selecting a membrane, therefore, one looks for a membrane displaying high intrinsic permeability for a certain component and low intrinsic permeability to other components of the mixture, i.e., high permeability and high selectivity. These properties are seldom found simultaneously in one polymer. This dilemma is an important factor in the development of competitive membrane processes for the industrial separation of gases or liquids and medical treatment of wounds.

In practice, the membrane separation of gases or liquids is effected by passing a feed stream of the gas or liquid mixture to be separated over the surface of a membrane, allowing only a portion of the gas or liquid to permeate and either recycling the non-permeating gas or liquid (reffinate) or routing it to another permeation cell. The driving force for the permeation of the gas or liquid is the partial pressure difference across the membrane, maintained, for example, by either hydrostatic pressure on the feed side or by a vacuum on the product side of the cell or by both. Each pass of the mixture through a membrane is referred to as a stage of the separation, and the fraction permeated is referred to as the stage cut. An increase in the number of stages will result in a product of higher purity than that obtained in a single stage process. The cost of recompressing the gas at each stage, as well as the cost of additional membrane modules, however, is often prohibitive. The flux of the gas mixture to be separated may be increased by increasing the pressure difference across the membrane or by reducing the thickness of the membrane. In addition, in straight forward gas production processes, the design of the permeator will also influence the flux as well as the practically attainable selectivity of the membrane.

One of the common approaches to maximizing membrane flux is the reduction of its resistance, i.e., the thickness of the membrane itself. This approach, however, is fraught with two serious difficulties that were overcome only within the last twenty years. Gas and some liquid molecules are so small that pinholes, or even microscopic imperfections in the membrane surface, are enough to render the membrane useless for mixture separations. The casting of defect free ultrathin membranes by conventional casting techniques is exceedingly difficult. Irregularities in the surface upon which the membrane is cast will be incorporated into the membrane's structure which for ultrathin membranes may be of a magnitude in size comparable to the thickness of the membrane. These irregularities may result in pinholes through which the bulk flow of gases may occur, thus leading to a loss of permselectivity. Secondly, ultrathin membranes are extremely fragile and are often unable to withstand the pressures to which they must be subjected in membrane separation processes.

Several patents have issued disclosing various laminate or composite structures for gas permeable membranes produced by sandwiching a polymer membrane with higher gas separation coefficient between permeable silicone polymers or vice versa. For instance, see Sugata, et al., Jap. Pat. O.P.I No. 301/84, published January 5, 1984; Saito, et al., Jap. Pat. O.P.I. No. 223,411/83, published December 26, 1983; Hirose, U.S. Pat. No. 4,470,831, issued September, 1984; Lundstrum, U.S. Pat. No. 3,767,737, issued October, 1973.

In the early 1960's, methods were developed by Weyenberg and Findlay to form stable polymeric emulsions from siloxane oligimers via emulsion polymerization. (See D. Weyenberg, D. Findlay, U.S. Pat. No. 3,294,725 issued December 27, 1966). Although these materials found utility in applications requiring aqueous deposition of polysiloxane oils, it was not until the invention of Johnson, Saam, and Schmidt that practical routes to a true silicone latex became available. (See Johnson, et al., U.S. Pat. No. 4,221,688 issued September 9, 1980.)

Two patents issued to Traver (U.S. patents 4,518,727 and 4,529,758) address the formation of water based silicones. U.S. Patent Number 4,518,727, issued May 21, 1985, teaches a method of forming water based silicone resin emulsions from certain siloxane materials and anionic cellulosic emulsification agents. U.S. Patent Number 4,529,758, issued July 16, 1985, teaches a method of forming silicone water based resin dispersions from organopolysiloxane resin compositions and cellulosic or vinyl addition polymers. Neither Traver patent addresses the instant invention of forming semipermeable composite membranes from the deposition of a silicone water based emulsion on porous synthetic or living substrates.

Henis and Tripodi, in U.S. Patent No. 4,230,463, issued October 28, 1980 demonstrated porous substrates coated with polymers in occluding contact. Their approach was to deposit a thin layer of high permeability polymer (such as silicone rubber) on the surface of a porous membrane displaying good intrinsic selectivity for one component of the gas mixture to be separated. By sealing the surface pores in this manner, it was found that much of the selectivity of the underlying porous polymer material is retained, yet the overall permeability remains quite high. Membranes consisting of two or more films laminated together are referred to as composite membranes. Analogous to the flow of current in an electrical circuit, the ultimate properties of these composite membranes depend upon the respective resistances of the component parts; in this case, the coating material, the substrate material, and the pores.

Browall, in U.S. Patent No. 3,980,456, issued September 14, 1976, described a method for sealing pinholes and covering imperfections in multi-layer composite membranes to be employed in gas separation processes. Browall described the use of an ultrathin membrane of very flexible, permeable polymer material, particularly identifying organopolysiloxane-polycarbonate copolymer membranes, to coat the outer surface of the composite membrane consisting of, for example, polyphenylene oxide/organopolysiloxane-polycarbonate copolymer. However, Browall deposited the flexible, permeable film from an organic solvent, the preferred solvent being 1,2,3-trichloropropane (TCP). The requirement of an organic solvent significantly limits the type and nature of the polymer substrates which can be utilized under the Browall patent.

Romesberg et al., in Canadian Patent number 738,350, issued July 12, 1966, disclosed a process for forming composite membranes from coagulated latexes and dispersions of film-forming solids of thermoplastic polymers by depositing the dispersions on the coagulated latex, drying and fusing the material. However, Romesberg et al. utilized no silicone water based emulsions, no cellulose acetate substrate, and no polysulfone microporous substrate.

U.S. patent number 4,244,849, issued January 13, 1981 to John Saam, teaches the preparation of an aqueous silicone emulsion which provides an elastomeric product upon removal of the water under ambient conditions. The emulsion described therein comprises a continuous water phase and an anionically stabilized dispersed silicone phase which is a graft copolymer of a hydroxyl endblocked polydiorganosiloxane and an alkali metal silicate.

One of the main applications for the polyorganosiloxane-coated polymers of the present invention is the separation of oxygen and nitrogen from air mixtures. Gas permeation through a rubbery membrane (such as polyorganosiloxane rubber) is controlled by molecular diffusion (the rate determining step) with solution equilibrium established between the penetrant in the gas phase and in solution at the membrane surface. In gas separation through glassy polymers at temperatures below Tg, the motions of the polymer chains are not sufficiently rapid to completely homogenize the penetrant's environment. Penetrant molecules can thus occupy microcavities of different sizes and with very different intrinsic mobilities.

The instant invention allows the use of organic solvent sensitive substrates, including porous membranes, utilized in the production of composite membranes for the separation of fluids, such as gases or liquids. This is accomplished by using a silicone water-based emulsion to produce a polyorganosiloxane coating for the protection of a hydrocarbon solvent-sensitive substrate, such as synthetic or living tissue, from subsequent damage being caused when coated with a solution of a polymer dissolved in a hydrocarbon solvent. As a further advancement, the use of a silicone-water based emulsion in the instant invention to coat the polymer substrate does not itself degrade the solvent sensitive substrate to which it is applied. Further, upon the removal of the aqueous phase of the emulsion and subsequent cure of the polyorganosiloxane, the silicone-water based emulsion produces a stable continuous, permeable polysiloxane film on the polymer substrate.

One application for the semipermeable composite membranes of the instant invention is the medical treatment of wounds to living tissues. Burn victims, for example, are at an extreme risk of suffering from both infection and severe dehydration as a result of the loss of large areas of skin. The preferred treatment would allow the skin to breathe to thereby enhance the healing process but would preclude moisture-borne infective agents. The composite membranes of the instant invention, possessing a hydrophobic silicone deposited from the silicone-water based emulsion, is expected to provide such a medical treatment. The resulting medical treatment device for wounds should be somewhat flexible, easily applied, and contain no solvent carriers which might adversely affect living tissues.

The instant invention relates to a method of forming a fluid separation membrane having an organic solvent-sensitive porous substrate coated by an essentially continuous thin film of an organopolysiloxane polymer, said method comprising

(A) coating the porous surface of an organic solvent sensitive organic polymeric material with an aqueous emulsion of a curable polyorganosiloxane, said emulsion being substantially free of any organic solvents having a tendency to decrease the porous structure of said porous substrate and having a sufficient solids content such that an essentially continuous film of polyorganosiloxane is formed over the surface of the porous surface after removal of the aqueous components of the emulsion,

(B) substantially removing the aqueous components of the emulsion by drying the emulsion to form a stable durable film, and

(C) curing the resulting film to form an essentially continuous film of a cured polyorganosiloxane polymer over the surface of the porous surface. The polyorganosiloxane polymer is said to "cure" when sufficient moisture evaporation and crosslinking reactions have occurred so as to change the liquid emulsion material into a rubbery, non-tacky, solid. The continuous thin film membranes of an organopolysiloxane polymer produced by the present invention are approximately 0.1 to 20.0 micrometers in thickness.

The present invention also relates to a method for producing a device for medically treating wounds to the skin, said method comprising

(A) applying to a porous barrier substrate a coating of a curable organopolysiloxane water based emulsion,

(B) removing the aqueous components of the emulsion by drying the emulsion to form a coating and

(C) curing said coating.

The instant invention also relates to skin wound treatment devices produced by this method. Wounds to the skin may also be treated by applying thereto composite membranes to the instant invention comprising a porous substrate which has been coated on both sides with a cured film produced by the deposition of a silicone water based emulsion material, followed by the evaporation of the aqueous portion of the emulsion and cure of the organopolysiloxane.

The present invention also relates to the coating of porous surfaces bearing the configuration of a tube or hollow fiber through which can be passed fluid mixtures for separation or controlled release. The configuration of the porous surface to be coated by the instant invention can also be a flat sheet.

In the present invention, composite membranes suitable for mixture separation were prepared by the application of a silicone (i.e., polyorganosiloxane) water based emulsion to the surface of a porous polysulfone substrate and other polymers such as cellulose, cellulose acetate, polyphenylene oxide, and polyacetonitrile . The silicone water based emulsions serviceable in the instant invention are limited only by the need to provide a cured continuous organopolysiloxane on the porous surface upon the evaporation of the aqueous components of the emulsion and subsequent crosslinking of the organopolysiloxane. Thus, the curable organopolysiloxanes serviceable in the emulsions used in the instant invention can be, for example, but are not limited to, dihydroxy dimethylpolysiloxane polymers, aminodimethylpolysiloxane polymers, trimethylsilyl dimethylpolysiloxane polymers, trifluoropropyl-containing polysiloxane polymers and phenylmethylpolysiloxane polymers.

Emulsion polymerization methods for making emulsions of polymers involve starting with low viscosity polymer precursors, i.e., monomers, or reactive oligomers, which are immiscible in water, a surfactant to stabilize the polymer precursor droplet in water, and a water soluble polymerization catalyst. These components are added to water, the mixture is stirred and polymerization is allowed to advance until the reaction is complete or the desired degree of polymerization is reached and a standard emulsion of the polymer is formed.

An example of an emulsion polymerization is taught in U.S. Patent No. 2,891,920 issued to Hyde et al., which shows a method for making aqueous emulsions of polydimethylsiloxane.

All known condensation or emulsion polymerization catalysts used in the emulsion polymerization of polyorganosiloxanes are useful in practicing the present invention method. Such catalysts include cationic and anionic species. The cationic catalysts include strong bases, for example, quaternary ammonium hydroxides of the formula $R_4NOH$ such as tallowtrimethylammonium hydroxide (the chloride salt is known as Arquad® T sold by Armak Company, Chicago, Illinois), and metal hydroxides such as sodium hydroxide. The art reveals a large number of cationic catalysts that work to condense siloxanes in the presence of water which are useful in practicing the invention.

Anionic condensation catalysts include strong mineral acids, aliphatically substituted benzenesulfonic acids and aliphatic sulfonic acids, but are not limited to such catalysts. Any catalyst that polymerizes polyorganosiloxanes in the presence of water can be used to practice the invention.

The surfactant used to produce the stable silicone water based emulsions is limited only by the need to provide a cured continuous organopolysiloxane on the porous surface upon the evaporation of the aqueous components of the emulsion and subsequent crosslinking of the organopolysiloxane. The stabilizing surfactant can be a cationic stabilizer (as, for example, when the silicone water based emulsion contains an amino-functional polysiloxane), an anionic stabilizer (as, for example, when the silicone water based emulsion contains a carboxy-functional polysiloxane), or a nonionic surfactant. Methods of manufacturing such polysiloxane emulsions are known in the art, as in, for example, U.S. Patent No. 4,244,844.

The coating emulsions employed in the preparation of the membranes of the instant invention were obtained from Dow Corning Corporation, Midland, Michigan. The preferred material is a polysiloxane emulsion of approximately 30 to 50% by weight solids. It is prepared by the acid catalyzed polymerization of low molecular weight dihydroxy dimethylpolysiloxane polymer, $HO[Si(CH_3)_2O]_xH$, wherein x is at least 7, in the presence of a surfactant, to form a high molecular weight silicone emulsion. The preferred acid catalysts include hydrogen chloride, the dodecylbenzenesulfonic acid while the surfactant can be the salt of the sulfonic acid used in the emulsion polymerization. The alkali metal salts of the sulfonic acids are preferred, particularly the sodium salts. The sulfonic acid can be illustrated by benzenesulfonic acids, aliphatically substituted naphthalene sulfonic acids, aliphatic sulfonic acids, silylalkylsulfonic acids and aliphatically substituted diphenylethersulfonic acids. In the preferred embodiment of the invention, the emulsion is of low viscosity (for example, approximately 600 centipoise) but a wide viscosity range is acceptable. The emulsion is translucent. The thickness of the cured silicone coating upon the porous substrate is dependent upon the viscosity and silicone concentration of the silicon water based emulsion.

It has also been observed that thinner, pinhole-free organopolysiloxane films can be deposited on the porous surface if the emulsions exhibit silicon oil particle sizes of approximately 50 to 200 Å (0.02 microns). So called microemulsions are capable of being diluted to lower solids content with water than are conventional silicone emulsions (of particle size approximately 3000 Å) without breaking the emulsion. Microemulsions are mixtures of oil and water where the particle size of the resulting droplets is small enough so the resulting mixture is clear. Because of their clarity, microemulsions are distinguishable from standard, opaque emulsions. Microemulsions of polydiorganosiloxane and water offer a number of advantages over standard emulsions. The clarity of the mixtures is advantageous in cosmetic applications, and the reduced particle size of the droplets is advantageous where it is necessary to deposit particles in small pores or as thin films. Microemulsions are also more temperature, dilution, and formulation stable than are standard emulsions. Methods for making microemulsions of polyorganosiloxane are known in the literature. See, for example, U.S. patent No. 3,433,780 issued to Cekada on March 18, 1982; U.S. Patent nos. 3,975,294 and 4,052,331 both issued to Dumoulin on August 17, 1976 and October 4, 1977, respectively; and U.S. Patent No. 4,146,499 issued on March 21, 1979 to Rosano; and U.S. Application Serial Number 809,090 filed December 12, 1985 Methods for Making Polydiorganosiloxane Microemulsions, D. Graiver and O. Tanaka. The ability to further dilute the silicone water based microemulsion provides thinner, yet still continuous, curable organopolysiloxane films serviceable in the composite membranes of the instant invention.

The preferred specific gravity of the emulsion is in the range of approximately 1.0 to 1.6 and the preferred pH is in the range of approximately 7 to 11.6. Upon removal of the water phase and crosslinking the organopolysiloxane polymer, a cured silicone rubber is produced which has a Shore A durometer reading of approximately 40 (ASTM D2240), and tensile strength of approximately 250 psi (ASTM D412). The silicone is said to "cure" when sufficient moisture evaporation and crosslinking reactions have occurred so as to change the liquid emulsion material into a rubbery, non-tacky, solid. In order to produce a cured organopolysiloxane cast film, it is known in the art that a crosslinker such as a silane or colloidal silica must be present in the silicone water based emulsion in addition to an emulsion polymerization catalyst such as a dialkyltin dicarboxylate. See, for example, U.S. Patent No. 4,228,054 issued October 14, 1980, to Ona et al., which teaches the use of organofunctional silanes as crosslinkers in organopolysiloxane emulsions. Absent the crosslinker, a gum rather than a cured film is produced upon evaporation of the water. See Johnson et al., U.S. Patent No. 4,221,688, issued September 9, 1980, for the use of colloidal silica in silicone water based emulsions. In the instant invention, the crosslinker is limited only by the need to provide a continuous cured film of organopolysiloxane on the porous surface upon the evaporation of the aqueous components of the emulsion and subsequent crosslinking of the organopolysiloxane.

Organopolysiloxanes are particularly well suited as top coats over the less permeable polysulfone, cellulose acetate and polyphenylene oxide glassy substrates or as a middle layer in a composite membrane. A diffusing gas or liquid, which has penetrated the permeable organopolysiloxane film, can diffuse either directly through the organopolysiloxane film to the glassy substrate beneath or can be channeled laterally through the organopolysiloxane film, which acts much like a conduit, until a surface pore in the substrate beneath is encountered. Therefore, the higher permeability of the organopolysiloxane film results in enhancement of the flux of the composite membrane by increasing the effective surface area of the separating film.

One of the methods of producing thin membranes is to dilute the emulsion system before casting the film or membrane. Thus, due to the viscosity of the silicone water based emulsion material, the emulsions used in the instant invention are preferably, but not necessarily, further diluted in distilled water to, for example, 25% to 12.5% by weight solids, before being applied to the porous substrates. The latter dilution results in membranes with significantly lower separation factors of in the range of 1.3 to 1.6 instead of the common value of approximately 2.2 when undiluted. The microemulsions can be diluted even further without breaking the emulsion. The silicone water based emulsion dries to form a film which rapidly undergoes crosslinking reactions to form a cured, flexible, porous coating at ambient conditions or at elevated temperatures up to 100 degrees Centigrade. Because there is no hydrocarbon solvent carrier to dissolve and/or degrade the porous substrate, the composite membranes of the instant invention provide a significant improvement over the prior art.

7

Polysulfone, cellulose acetate, and polyphenylene oxide composite membranes were prepared by the Polymer Research Institute of the State University of New York, Syracuse, New York. Polysulfone was prepared by the Friedel-Crafts condensation of phenyl sulfonyl chloride. Cellulose acetate polymers were obtained from Eastman Chemical Products, Inc., Kingsport, Tennessee as cellulose acetate No. 4655 (39.4% acetyl content). Polyphenylene oxide was obtained from General Electric Company, Schenectedy, New York under the name "PPO" or" polyphenylene oxide." Kraft cellulose paper was obtained from Dow Corning Corporation, Midland, Michigan.

The parameter termed "actual separation factor" was determined by analyzing the gas product permeated at a very low stage cut (<2%). The term "ideal separation factor" refers to the ratio of permeabilities measured by allowing pure gas to permeate through the membrane at a specified pressure. Dry compressed air was fed to the cell at 10 to 100 psi via a feed line. The low pressure side of the membrane was either maintained at atmospheric pressure or connected to a vacuum pump. Flow rates for the gas were measured using a Brooks Sho Rate Rotameter.

The rate of permeation of the gas mixture through the membrane is commonly described as follows:

$$\text{Flux } (cm^3/sec \cdot cm^2) = \frac{(\overline{P}) \ (\Delta p) \ x \ A}{l}$$

where $\overline{P}$ = permeability coefficient
$l$ = thickness of material permeated, (cm)
$A$ = surface area, (cm$^2$)
$\Delta p$ = pressure differential across the membrane, (psi)

The gas mixtures were analyzed by an F and M Gas Chromatograph (Model 720) having a thermal conductivity detector. The areas of the peaks on the gas chromatograph strip chart recording were calculated by multiplying the height times the width at one half the height. The gas composition was calculated by dividing the total area of the oxygen and nitrogen peaks by the area of the peak corresponding to each specific gas. The separation factor $O_2/N_2$ was calculated by the formula

$$SF_{O_2/N_2} = \frac{(A_{O_2}) \ (A_{N_2}) \ \text{Product}}{(A_{O_2}) \ (A_{N_2}) \ \text{Feed}}$$

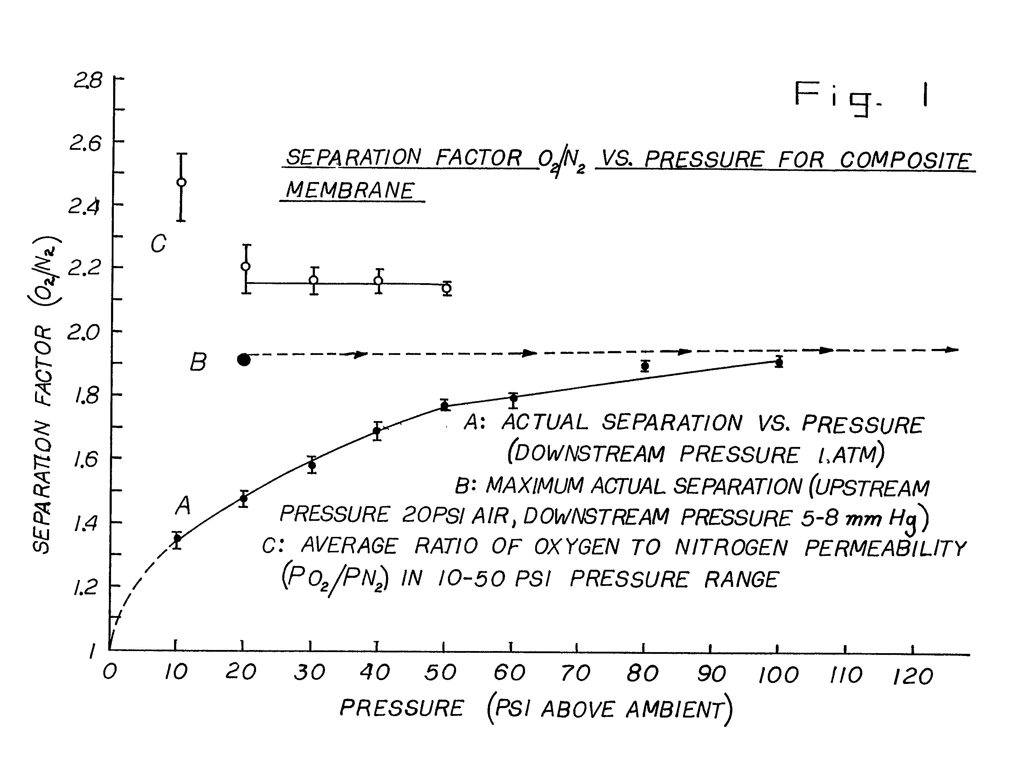

The curve labeled "A" of the graph in FIG. 1 plotting separation factor vs. air pressure for the instant invention asymptotically approaches the separation factor of 1.91-1.92 calculated for a membrane exposed to a downstream pressure of about 5 to 8 mm Hg. This corresponds to a pressure ratio of 130-200 to 1 between the upstream and downstream sides of the membrane. (See Figure 1) Oxygen enrichment was easily attained with the membranes of the instant invention from the normal 20% oxygen found in air up to 32-34% oxygen by pressurizing the feed gas with about 50 psi. In FIG. 1 line C shows: deal separation factors as obtained from the permeability ratios of the pure gases measured independently.

Based on a silicone layer thickness of $3.84 \times 10^{-3}$ cm, (determined by measuring the polysulfone thickness and composite membrane thickness by micrometer), the average oxygen and nitrogen permeabilities for the composite membrane of the instant invention were $1015 \times 10^{-10}$ and $447 \times 10^{-10}$, respectively, measured in units of
$cm^3 \ sec^{-1} \cdot cm \ cm^{-2} \ (cmHg)^{-1}$.

A common unit of measurement for permeability coefficients is the Barrer, where one Barrer equals $1cm^3 \cdot sec^{-1} \cdot cm^{-1} \ (cmHg) \times 10^{-10}$.

The cellulose acetate membrane coated with the silicone water based emulsion membrane was tested for permeability with a gas pressure of 100 psi on the upstream side and 1 atmosphere of pressure on the downstream side. The oxygen and nitrogen permeabilities observed were 6.82 and $2.18 \times 10^{-8} \ cm^3 \ sec^{-1} \cdot cm \ cm^{-2} \ (cmHg)^{-1}$, respectively. The ideal separation factor from these measurements is 3.13. By the present invention, the application of silicone water based emulsion coatings to microporous cellulose acetate, at membrane thicknesses of from 1 to 2 microns, produced oxygen and nitrogen permeabilities of $P(O_2)$ 603 Barrer and $P(N_2)$ 290 Barrer, respectively.

By this invention, composite membranes suitable for separation of fluids are produced on porous and other polymer substrates without adversely affecting the supporting material, without requiring a hydrocarbon solvent, and without requiring a subsequent curing treatment with a crosslinking agent. The composite membranes are useful for separating and concentrating the components of fluid mixtures by passing the mixtures through the membranes.

In the following examples, asymmetric cellulose acetate membranes were prepared by dissolving 20% by weight cellulose acetate in a mixture of 60 parts acetone and 40 parts formamide. This solution was centrifuged to remove suspended particles and was cast on a glass plate to a casting thickness of 0.020 inches. After an evaporation time of 25 to 60 seconds, the plate was submerged in a bath of ice water at approximately 4 degrees Centigrade to achieve coagulation. Membranes were prepared with evaporation times of 25, 40, and 60 seconds. In order to dry the membrane without collapsing the porous structure, the membranes were transferred to a bath containing a 25% solution of glycerol in water and allowed to stand overnight.

Example 1

Membranes prepared as described above were coated with a silicone water based emulsion material (50% solids of high molecular weight silicone polymer in water produced by the acid catalyzed polymerization of low molecular weight dihydroxy dimethyl siloxane polymer). The specific gravity of the emulsion was approximately 1.02 and the pH was 11.2. Due to the viscosity of the silicone water based emulsion material, the emulsion was diluted in distilled water to 25% by weight solids before being applied to the porous cellulose acetate substrate. The silicone water based emulsion material was cured by a tin catalyzed crosslinking reaction (as taught in Saam U.S. Patent No. 4,224,849) followed by the removal of the water phase of the emulsion by evaporation at room temperature. The silicone is said to "cure" when sufficient crosslinking reactions and moisture evaporation have occurred so as to change the liquid emulsion material into a rubbery, non-tacky, solid. The resulting composite membranes are approximately 0.1 to 20.0 micrometers in thickness.

Example 2

A 0.2% solution of polyphenylene oxide (PPO) in a 10 to 1 by weight mixture of carbon tetrachloride and chloroform, respectively, was coated, using a flow coat casting blade, onto the surface of a polysulfone/silicone water based emulsion composite membrane prepared by the method of Example 1, with the exception of using polysulfone rather than cellulose acetate as the porous substrate. The coating was dried and allowed to cure as in Example 1.

Example 3

A polysulfone support membrane was coated with the silicone water based emulsion material, described in Example 1, by the following procedure. The silicone water based emulsion material was stirred to insure homogeneity, diluted to 50% by weight (or 25% total solids) in distilled water. The emulsion was then flow coated over the surface of the polysulfone during a residence time of approximately 30 seconds. The excess silicone water based emulsion material was then poured off and the coating allowed to dry at room temperature for approximately two hours. The silicone water based emulsion material layer crosslinked spontaneously during the drying process resulting in an ultrathin cured polysiloxane coating upon the porous substrate. The composite membranes thus produced yielded separation factors of 2.1 for an oxygen/nitrogen (air) mixture.

Example 4

The silicone water based emulsion material described in Example 1 was diluted to 35% weigh solids with distilled water and the emulsion was spread over polysulfone porous support substrate. This yielded a 0.5 micron thick crosslinked polysiloxane layer upon the evaporation of the aqueous components of the emulsion. The resulting composite membrane was tested for separation of oxygen/nitrogen (air composition) and produced oxygen enriched air of 35.1% oxygen. The ultrathin layer yielded high fluxes which corresponded to permeability coefficients of 600 ± 20 Barrer for oxygen

Example 5

Silicone water based emulsion was sucked into the porous surface of a polysulfone substrate by means of applying a 500 mmHg vacuum to the opposite side of the polysulfone. The surface of the polysulfone was washed with water and allowed to air dry, to allow curing of the polysiloxane. When tested for the separation of air (20% oxygen), enriched air of 35% oxygen was produced by passing air through the membrane. The flux was equivalent to the flux that would have been obtained from a 0.2 micron thick polydimethylsiloxane layer with a permeability coefficient of 580 Barrer for oxygen.

**Claims**

1. A method for forming a fluid separation membrane having an organic solvent-sensitive porous surface coated by an essentially continuous thin film of an organopolysiloxane polymer, said method comprising

(A) coating the porous surface of an organic solvent sensitive organic polymeric material with an aqueous emulsion of a curable polyorganosiloxane, said emulsion being substantially free of any organic solvents having a tendency to decrease the porous structure of said porous surface and having a sufficient solids content such that an essentially continuous film of polyorganosiloxane is formed over the surface of the porous surface after removal of the aqueous components of the emulsion,

(B) substantially removing the aqueous components of the emulsion by drying the emulsion to form a stable, durable film, and

(C) curing the resulting film to form an essentially continuous film of a cured polyorganosiloxane polymer over the surface of the porous surface.

2. The method as claimed in claim 1 wherein the fluid separation membrane has a thickness in the range of 0.1 to 20.0 microns.

3. The method as claimed in claim 1 wherein the porous surface is selected from the group consisting of polysulfone, cellulose, cellulose acetate, cellulose esters, polyacetonitrile and polyphenylene oxide.

4. The method as claimed in claim 1 wherein the emulsion is formed by emulsifying a low molecular weight curable organopolysiloxane polymer, water, surfactant, and an acid catalyst.

5. A method as claimed in claim 1 wherein the curable organopolysiloxane polymer is $HO[Si(CH_3)_2O]_xH$ wherein x is at least 7.

6. A method as claimed in claim 4 wherein the acid catalyst is selected from the group consisting of hydrochloric acid, and dodecylbenzenesulfonic acid.

7. A method as claimed in claim 1 wherein the porous surface has the configuration of a flat sheet.

8. A method as claimed in claim 1 wherein the porous surface has the configuration of a tube.

9. A method as claimed in claim 1 wherein the porous surface is a hollow fiber.

10. A method as claimed in claim 1 wherein the aqueous emulsion is a macro emulsion wherein the polyorganosiloxane droplets are approximately 200 to 3000 Angstroms in size.

11. A method as claimed in claim 1 wherein the aqueous emulsion is a microemulsion wherein the polyogranosiloxane droplets are approximately 50 to 200 Angstroms in size.

12. The method as claimed in claim 1 wherein the film produced by drying the emulsion is cured at ambient conditions.

13. The method as claimed in claim 1 wherein the film produced by drying the emulsion is cured at elevated temperatures.

14. A membrane obtained by the method of any of claims 1, 2, 3, 4, 5, 10, 11, 12 or 13.

15. A membrane obtained by the method of claim 1 wherein the porous surface is coated with the emulsion on both sides.

16. A method for producing a device for medically treating wounds to the skin, said method comprising (A) applying to a porous barrier substrate a coating of a curable organopolysiloxane water based emulsion, (B) removing the aqueous components of the emulsion by drying the emulsion to form a coating, and (C) curing said coating.

17. A device produced by the method of claim 16 for medically treating wounds to the skin.

Fig. 1

SEPARATION FACTOR $O_2/N_2$ VS. PRESSURE FOR COMPOSITE MEMBRANE

A: ACTUAL SEPARATION VS. PRESSURE (DOWNSTREAM PRESSURE 1.ATM)

B: MAXIMUM ACTUAL SEPARATION (UPSTREAM PRESSURE 20PSI AIR, DOWNSTREAM PRESSURE 5-8 mm Hg)

C: AVERAGE RATIO OF OXYGEN TO NITROGEN PERMEABILITY $(PO_2/PN_2)$ IN 10-50 PSI PRESSURE RANGE

SEPARATION FACTOR $(O_2/N_2)$

PRESSURE (PSI ABOVE AMBIENT)

0 242 069